# EUROPÄISCHE PATENTSCHRIFT

(11) **EP 2 205 165 B1**
(45) Veröffentlichungstag und Bekanntmachung des Hinweises auf die Patenterteilung: **30.03.2011**
(21) Anmeldenummer: 08801852.8
(22) Anmeldetag: 04.09.2008
(51) Int. Cl.: A61B 17/15

(54) **SÄGELEHRE ZUM DURCHFÜHREN EINER KNOCHENRESEKTION**
SAW GAUGE FOR PERFORMING A BONE RESECTION
GABARIT DE SCIAGE PERMETTANT D'EFFECTUER UNE RÉSECTION OSSEUSE

(30) Priorität: 04.09.2007 DE 202007012383 U
(43) Veröffentlichungstag der Anmeldung: 14.07.2010
(73) Patentinhaber: Brehm, Peter, 91085 Weisendorf (DE)
(72) Erfinder: Brehm, Peter, 91085 Weisendorf (DE)
(74) Vertreter: Grünecker, Kinkeldey, Stockmair & Schwanhäusser Anwaltssozietät
(86) Internationale Anmeldenummer: PCT/EP2008/007257
(87) Internationale Veröffentlichungsnummer: WO 2009/030484

(56) Entgegenhaltungen:
- EP-A- 0 366 488
- EP-A- 1 444 956
- EP-A- 1 444 957
- EP-A- 1 665 992
- US-A1- 2006 235 290
- US-A1- 2007 173 849

## Beschreibung

Die vorliegende Erfindung bezieht sich auf eine Sägelehre zum Durchführen einer Knochenresektion, insbesondere auf eine Sägelehre, in der ein Winkel zwischen einem Befestigungsabschnitt und einem Sägeführungsabschnitt verstellbar ist.

In der Knieendoprothetik kommen Implantate zum Einsatz, die aus je einer metallischen Komponente für den Unterschenkel (Tibia) und dem Oberschenkel (Femur) bestehen. Diese wirken über ein Meniskallager, das beispielsweise Komponenten aus Polyethylen umfassen kann, als künstliches Gelenk zusammen. Für die metallischen Komponenten kommen neben Kobalt-Chromlegierungen auch Titanlegierungen und Keramik zum Einsatz. Dem Innen- und Außenmeniskus eines natürlichen Kniegelenks nachempfunden, wirkt das künstliche Gelenk entsprechend über eine mediale und eine laterale Kondyle zusammen.

Die Lebensdauer des künstlichen Gelenkersatzes wird in erster Linie durch den Verschleiß der Komponenten aus Polyethylen begrenzt. Abriebspartikel dieser Komponenten, insbesondere Polyethylenpartikel, können umliegendes Knochengewebe zerstören und werden hauptsächlich für frühzeitige Implantatiockerungen verantwortlich gemacht. Der Wechsel eines gelockerten Implantats durch eine Operation ist medizinisch aufwendig und mit Belastungen für den Patienten verbunden. Es wurde festgestellt, dass zur Reduzierung des Verschleißes des Meniskallagers einer gleichmäßigen Lastübertragung über die mediale und die laterale Kondyle bzw. einer korrekten Ausrichtung der metallischen Komponenten eine zentrale Bedeutung zukommt.

Bei der Implantation einer Knieendoprothese wird in der Regel mit der tibiaseitigen Ausrichtung und Bearbeitung begonnen, um darauf aufbauend die gewünschte Beinachse einzustellen. Die Tibiakomponente besteht aus einem Plateau mit einem oder mehreren sich in den Unterschenkel erstreckenden Zapfen zur Verankerung im Knochen. Um dem Tibiaplateau eine ebene Auflagefläche zu bieten, muss ein Stück des Knochens entfernt werden. Zugunsten einer lastgerechten Einleitung der Kräfte von der Tibiakomponente der Knieendoprothese, die ein relativ hohes Elastizitätsmodul aufweisen kann, in den Knochen, der einen geringeren Elastizitätsmodul aufweisen kann, wird eine Auflageebene angestrebt, die sich mindestens von medial nach lateral senkrecht zur Beinachse erstreckt.

Die Abtragung des Knochens kann mit Hilfe einer oszillierenden Säge erfolgen. Zur Führung des Sägeblatts kann eine Sägelehre an dem Tibiaknochen befestigt werden. In Sägelehren nach dem Stand der Technik wird die Befestigung mit Hilfe von zwei Nägeln bzw. Schrauben vorgenommen, die durch parallele Bohrungen in der Sägelehre geführt werden. Die Sägelehre kann nach dem Tibiaschnitt abgenommen werden, während die Nägel bzw. Schrauben als Referenz für Nachresektionen vorübergehend im Knochen belassen werden können.

Durch die Anatomie der Tibia gegeben, können die Nägel bzw. Schrauben aus einer anderen als der senkrechten Richtung auf der Knochenoberfläche auftreffen. Dies kann dazu führen, dass die vorher nach den oben genannten Kriterien ausgerichtete Sägelehre die Tendenz aufweist, ihre optimale Ausrichtung bei der Befestigung zu verlieren. Deshalb werden Sägelehren mit vorgegebenem Korrekturwinkel, z.B. mit einem Korrekturwinkel von 2°, vorgesehen. Bei größeren Winkelabweichungen muss eine der Befestigungsschrauben neu platziert werden, was zu instabilen Verhältnissen führen und das gesamte Behandlungsergebnis verschlechtem kann.

Die Druckschrift 2007/173849 A1, von der der Oberbegriff des Anspruchs 1 abgeleitet wird, beschreibt eine Sägelehre zum Durchführen einer Knochenresektion. Diese bekannte Sägelehre umfasst einen Befestigungsabschnitt mit mehreren Bohrungen zur Aufnahme von.Befestigungsmitteln und einen Sägeführungsabschnitt, der wiederum ein Führungsmittel umfasst, das zum Führen eines Sägeblatts in einer Ebene geeignet ist. Zum Justieren des Varus-/Valguswinkels weist der Sägeführungsabschnitt eine Stellschraube auf.

Die Druckschrift DE10207035 A1 offenbart ebenfalls eine Sägelehre in Form einer Schablone zur Führung eines chirurgischen Bearbeitungswerkzeugs. Die Schablone kann mit drei Schrauben an einem Knochen befestigt werden. Die Schablone umfasst einen Führungsblock, der zwei geradlinige Führungsschlitze aufweist. Dabei kann das Sägeblatt einer oszillierenden Säge durch einen der Führungsschlitze hindurchgeschoben werden. Eine Winkelstellung des Führungsblocks kann mit Hilfe von zwei Stellschrauben eingestellt werden.

Es ist eine Aufgabe der vorliegenden Erfindung, eine Sägelehre bereitzustellen, die ein präziseres und zeitsparenderes Arbeiten erlaubt.

Gemäß der vorliegenden Erfindung umfasst eine Sägelehre zum Durchführen einer Knochenresektion einen Befestigungsabschnitt mit mehreren Bohrungen zur Aufnahme von Befestigungsmitteln, die für eine Befestigung des Befestigungsabschnitts an einem Knochen ausgebildet sind. Die Sägelehre umfasst femer einen Sägeführungsabschnitt, der ein Führungsmittel umfasst, das zum Führen eines Sägeblatts in einer Ebene geeignet ist. Der Sägeführungsabschnitt ist durch zwei Stellschrauben mit dem Befestigungsabschnitt verbunden. Das Führungsmittel ist zwischen den Stellschrauben angeordnet und die Stellschrauben weisen zueinander parallele Achsen auf. Ein Ende von jeder der Stellschrauben ist durch ein Gelenk mit dem Befestigungsabschnitt verbunden. Dadurch wird eine größere Beweglichkeit des Sägeführungsabschnitts relativ zum Befestigungsabschnitt ermöglicht, wodurch der Winkel zwischen dem Sägeführungsabschnitt und dem Befestigungsabschnitt in einem größeren Bereich eingestellt werden kann.

Durch Drehen an den Stellschrauben kann ein Winkel zwischen dem Befestigungsabschnitt und dem Sägeführungsabschnitt verstellt werden, wobei die Verstellung stufenlos erfolgen kann. Auch der Abstand zwischen dem Sägeführungsabschnitt und dem Befestigungsabschnitt kann zur Anpassung der Resektionshöhe verändert werden, z.B. durch gleichsinniges Verdrehen der beiden Stellschrauben. Durch die Verstellbarkeit des Winkels und des Abstands zwischen dem Befestigungsabschnitt und dem Sägeführungsabschnitt kann im Vergleich zu Sägelehren nach dem Stand der Technik, die einen vorgegebenen Korrekturwinkel aufweisen, eine präzisere Einstellung des Tibiaschnitts erfolgen. Durch die höhere Genauigkeit der Knochenresektion kann eine verbesserte Gesamtausrichtung der dem Patienten implantierten Knieendprothese erreicht werden, wodurch eine Verringerung des Verschleißes der Knieendoprothese und somit eine längere Standzeit der Prothese ermöglicht werden kann. Außerdem können Nachresektionen vermieden und damit eine zum Implantieren der Knieendprothese erforderliche Operationszeit verringert werden. Femer kann der Befestigungsabschnitt mit einer geringeren Präzision als der, die bei Verwendung einer Sägelehre nach dem Stand der Technik erforderlich ist, am Knochen des Patienten befestigt werden, und die Feinjustierung kann mit Hilfe der Stellschrauben erfolgen. Dadurch kann die Zeit, die zum Durchführen der Knochenresektion erforderlich ist, verringert werden. Außerdem wird durch die geringeren Anforderungen an die Genauigkeit der Befestigung des Befestigungsabschnitts eine Vereinfachung des Instrumentariums für die Ausrichtung des Befestigungsabschnitts ermöglicht, wodurch die Kosten für das Instrumentarium verringert werden können.

Geeigneterweise ist jede der Bohrungen zur Aufnahme eines Knochennagels oder einer Knochenschraube ausgebildet. Mit Hilfe von Knochennägeln oder Knochenschrauben kann eine stabile Befestigung des Befestigungsabschnitts an Knochen des Patienten erreicht werden.

Geeigneterweise sind die Bohrungen zueinander im Wesentlichen parallel.

Vorteilhafterweise weist der Sägeführungsabschnitt zwei Führungsfüße auf, die jeweils in Öffnungen im Befestigungsabschnitt eingreifen. Femer weist die Sägelehre zwei Feststellschrauben zum Arretieren der Führungsfüße im Befestigungsabschnitt auf. Mit Hilfe der Führungsfüße und der Feststellschrauben kann der Säugeführungsabschnitt nach der Einstellung des Winkels zwischen dem Sägeführungsabschnitt und dem Befestigungsabschnitt arretiert werden, wodurch eine unbeabsichtigte Verstellung des Winkels zwischen dem Sägeführungsabschnitt und dem Befestigungsabschnitt vermieden werden kann.

In einer Ausführungsform umfasst das Führungsmittel mehrere zueinander parallele Rundstäbe. Bei der Knochenresektion kann das Sägeblatt zwischen den Rundstäben hindurchgesteckt werden, wobei die Rundstäbe eine unbeabsichtigte Verkippung des Sägeblatts verhindern.

Gemäß einer anderen Ausführungsform umfasst das Führungsmittel einen im Sägeführungsabschnitt ausgebildeten Schlitz. Bei der Knochenresektion kann das Sägeblatt durch den Schlitz gesteckt werden, wobei der Schlitz eine Bewegung des Sägeblatts in einer Ebene erlaubt und eine Verkippung des Sägeblatts gegen die Ebene verhindert.

Gemäß einer weiteren Ausführungsform umfasst das Führungsmittel eine ebene Auflagefläche am Sägeführungsabschnitt. Eine Ausrichtung des Sägeblatts in einer Ebene kann durch Auflegen des Sägeblatts auf der Auflagefläche erzielt werden.

Ausführungsformen der vorliegenden Erfindung werden mit Bezug auf die Fig. 1 bis 3 beschrieben. Es zeigen:
- Fig. 1: eine schematische Perspektivansicht einer Sägelehre gemäß einer Aus- führungsform der vorliegenden Erfindung;
- Fig. 2A bis 2C: Ansichten einer Sägelehre gemäß der vorliegenden Erfindung in Stadien eines Verfahrens zum Durchführen einer Knochenresektion; und
- Fig. 3: eine schematische Perspektivansicht einer Sägelehre gemäß einer weite- ren Ausführungsform der vorliegenden Erfindung.

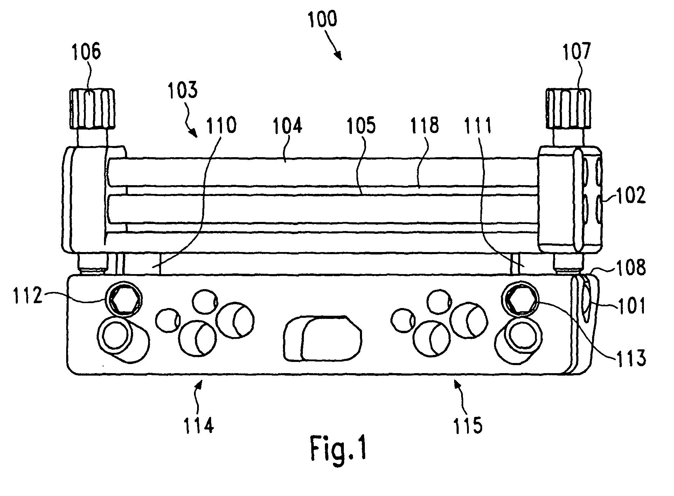

Fig. 1 zeigt eine schematische Perspektivansicht einer Sägelehre 100 gemäß einer Ausführungsform der vorliegenden Erfindung.

Die Sägelehre 100 umfasst einen Befestigungsabschnitt 101 und einen Sägeführungsabschnitt 102. Im Befestigungsabschnitt 101 sind mehrere erste Bohrungen 114 und mehrere zweite Bohrungen 115 vorgesehen. Der Befestigungsabschnitt 101 kann an einem Knochen eines Patienten befestigt werden, indem durch mindestens eine der ersten Bohrungen 114 und mindestens eine der zweiten Bohrungen 115 ein Knochennagel und/oder eine Knochenschraube gesteckt und in den Knochen des Patienten eingeschlagen bzw. eingedreht wird.

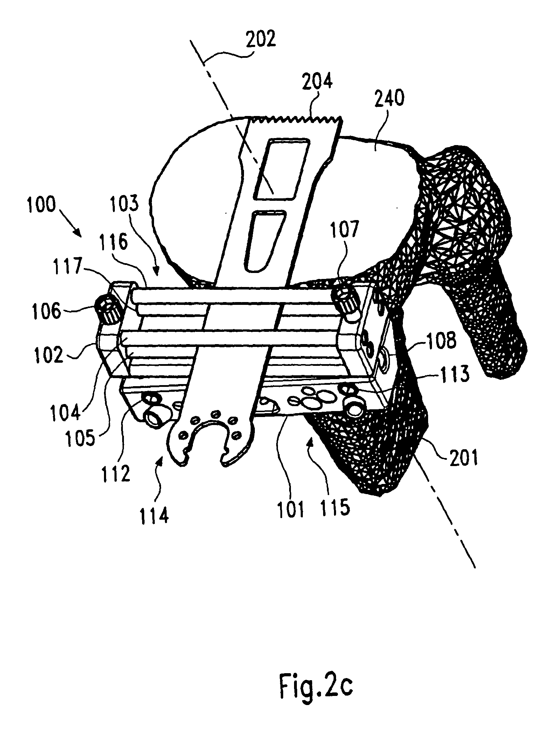

Der Sägeführungsabschnitt 102 weist ein Führungsmittel 103 auf. Dieses umfasst einen ersten Rundstab 104 und einen zweiten Rundstab 105. Zwischen dem ersten Rundstab 104 und dem zweiten Rundstab 105 befindet sich eine Lücke 118, die eine Breite aufweisen kann, die ungefähr gleich groß oder größer als eine Dicke eines Sägeblatts einer Knochensäge ist. Somit kann ein Sägeblatt zwischen den Rundstäben 104, 105 hindurchgesteckt werden. Zusätzlich zu den Rundstäben 104, 105 können weitere Rundstäbe 116, 117 (Fig. 2C) vorgesehen sein. Durch die Rundstäbe 104, 105, 116, 117 wird die Beweglichkeit des Sägeblatts im wesentlichen auf eine Ebene eingeschränkt, die durch die Anordnung der Rundstäbe 104, 105, 116, 117 vorgegeben ist.

Der Sägeführungsabschnitt 102 ist durch eine erste Stellschraube 106 und eine zweite Stellschraube 107 mit dem Befestigungsabschnitt 101 verbunden. Die Stellschraube 107 kann ein Außengewinde aufweisen, das in einem dazu passenden Innengewinde im Sägeführungsabschnitt 102 gedreht werden kann. Ein dem Kopf der Stellschraube 107 gegenüberliegendes Ende der Schraube kann derart mit dem Befestigungsabschnitt 101 verbunden sein, dass die Stellschraube 107 relativ zum Befestigungsabschnitt 101 um ihre Achse drehbar ist. Zu diesem Zweck kann im Befestigungsabschnitt 101 ein Gleitlager vorgesehen sein. Der Befestigungsabschnitt 101 kann femer ein Gelenk 108 aufweisen, das mit dem Gleitlager verbunden ist, so dass die Stellschraube 107 um eine zu ihrer Achse senkrechte Achse geschwenkt werden kann.

Ähnlich wie die Schraube 107 kann die Schraube 106 ein Außengewinde aufweisen, und der Sägeführungsabschnitt 102 kann ein zu diesem passendes Innengewinde umfassen. Im Befestigungsabschnitt 101 kann ein Lager, das eine Drehung der Schraube 106 um ihre Achse und ein Gelenk, das eine Drehung der Schraube 106 um eine zu ihrer Achse senkrechte Richtung ermöglicht, vorgesehen sein. Die Achsen der Stellschrauben 106, 107 können zueinander im wesentlichen parallel sein. Eine im wesentlichen parallele Ausrichtung der Achsen der Stellschrauben 106, 107 kann durch eine im wesentlichen parallele Ausrichtung der Innengewinde im Sägeführungsabschnitt 102 erzielt werden.

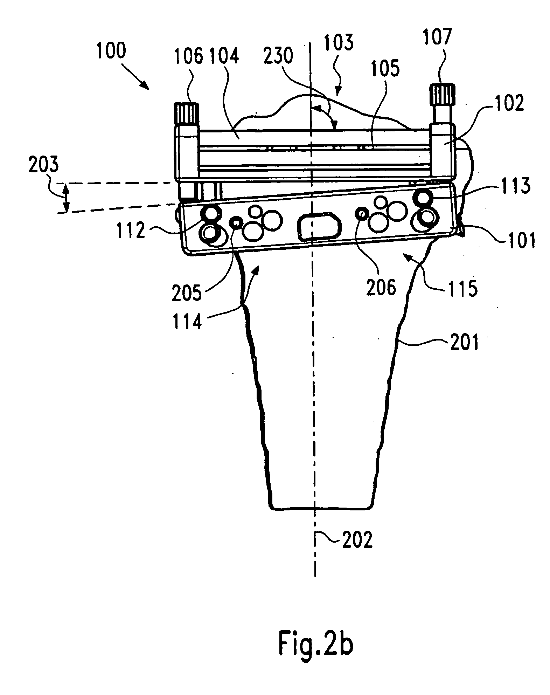

Durch Verstellen der Stellschrauben 106, 107 kann ein Winkel 203 (Fig. 2b) zwischen dem Sägeführungsabschnitt 102 und dem Befestigungsabschnitt 101 verstellt werden. Wenn der Befestigungsabschnitt 101 an einem Knochen eines Patienten befestigt ist, kann so ein Winkel zwischen der durch das Führungsmittel 103 vorgegebenen Ebene und dem Knochen verstellt werden. Dadurch kann beim Einsetzen einer Knieendoprothese ein Varus- bzw. Valguswinkel des Beins des Patienten justiert werden.

Mit Hilfe der Stellschrauben 106, 107 kann außer dem Winkel 203 auch der Abstand zwischen dem Sägeführungsabschnitt 102 und dem Befestigungsabschnitt 101 verändert werden. In Ausführungsformen, in denen die Stellschrauben 106, 107 Gewinde mit gleichem Drehsinn aufweisen, kann dies durch gleichsinniges Verdrehen der Stellschrauben 106, 107 geschehen, während der Winkel 203 durch gegensinniges Verdrehen der Stellschrauben 106, 107 eingestellt werden kann.

Der Sägeführungsabschnitt 102 kann einen ersten Führungsfuß 110 und einen zweiten Führungsfuß 111 aufweisen, die am Sägeführungsabschnitt 102 befestigt sind. Die Führungsfüße 110, 111 greifen jeweils in eine Öffnung (nicht gezeigt) im Befestigungsabschnitt 101 ein. Durch Bohrungen im Befestigungsabschnitt 101 können Feststellschrauben 112, 113 in die Öffnungen im Befestigungsabschnitt geführt werden. Wenn die Feststellschraube 112 in den Befestigungsabschnitt 101 eingeschraubt wird, trifft sie auf den in die Öffnung im Befestigungsabschnitt 101 ragenden Teil des Führungsfußes 110. Somit kann durch Anziehen der Feststellschraube 112 der Führungsfuß 110 in der Öffnung im Befestigungsabschnitt 101 festgeklemmt werden. Entsprechend kann der Führungsfuß 111 durch Anziehen der Feststellschraube 113 im Befestigungsabschnitt 101 festgeklemmt werden. Dadurch kann eine Ausrichtung des Sägeführungsabschnitts 102 relativ zum Befestigungsabschnitt 101 fixiert werden.

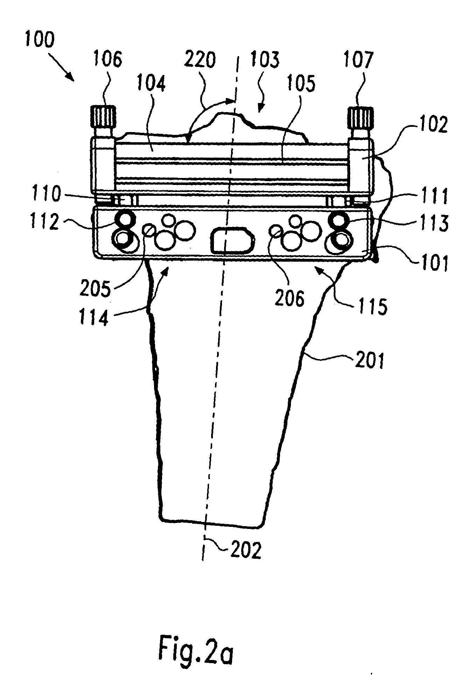

Fig. 2A zeigt eine schematische Ansicht eines Tibiaknochens 201 eines Patienten in einem ersten Stadium einer Knochenresektion, die mit Hilfe der Sägelehre 100 durchgeführt wird. Der Befestigungsabschnitt 101 wird mit Hilfe von Knochenschrauben 205, 206 am Tibiaknochen 201 des Patienten befestigt. In anderen Ausführungsformen können anstelle der Schrauben 205, 206 Knochennägel verwendet werden. Bei der Befestigung des Befestigungsabschnitts 101 wird die Sägelehre 100 derart ausgerichtet, dass die durch das Führungsmittel 103 definierte Ebene ungefähr senkrecht zu einer Längsachse 202 des Tibiaknochens 201 ist. Durch Ungenauigkeiten der Ausrichtung und/oder der Befestigung kann sich jedoch ein Winkel 220 zwischen der Ebene und der Längsachse 202 ergeben, der von einem rechten Winkel abweicht.

Fig. 2B zeigt eine schematische Ansicht des Tibiaknochens 201 mit der Sägelehre 100 in einem späteren Stadium des Verfahrens zum Durchführen einer Knochenresektion.

Nachdem die Sägelehre 100 am Tibiaknochen 201 befestigt wurde, wird durch Verstellen der Stellschrauben 106, 107 ein Winkel 203 zwischen dem Sägeführungsabschnitt 102 und dem Befestigungsabschnitt 101 justiert. Dadurch kann zwischen der durch das Führungsmittel 103 definierten Ebene und der Achse 202 des Tibiaknochens 201 ein Winkel 230 eingestellt werden, der im Wesentlichen gleich einem rechten Winkel ist. Anschließend kann der Sägeführungsabschnitt 102 durch Anziehen der Feststellschrauben 112, 113 fixiert werden, um eine unerwünschte Veränderung des Winkels 230 zu vermeiden.

Fig. 2C zeigt eine schematische Perspektivansicht der an dem Tibiaknochen 201 befestigten Sägelehre 100 in einem späteren Stadium des Verfahrens zum Durchführen einer Knochenresektion.

Nachdem der Winkel zwischen der durch das Führungsmittel 103 definierten Ebene und der Achse 202 justiert wurde und der Sägeführungsabschnitt 102 mit Hilfe der Feststellschrauben 112, 113 fixiert wurde, kann ein Sägeblatt 204 einer Knochensäge zwischen den Rundstäben 104, 105, 116, 117 des Führungsmittels 103 durchgesteckt werden, und mit Hilfe eines Antriebs in Bewegung versetzt werden. Knochensägen und Antriebe für Knochensägen sind den Fachleuten bekannt. Beispielsweise kann das Sägeblatt 204 parallel zu den Rundstäben 104, 105, 116, 117 hin- und herbewegt werden, so dass das Sägeblatt 204 eine oszillierende Bewegung ausführt. Durch das Sägeblatt kann ein Stück des Tibiaknochens 201 oberhalb der durch das Führungsmittel 103 definierten Ebene entfernt werden, wodurch man eine im Wesentlichen ebene Auflagefläche 240 auf dem Tibiaknochen 201 erhält. Da die durch das Führungsmittel 103 definierte Ebene im Wesentlichen senkrecht zur Längsachse 202 des Tibiaknochens 201 ist, kann die Auflagefläche 240 im Wesentlichen senkrecht zur Längsachse 202 des Tibiaknochens 201 ausgerichtet werden.

Anschließend kann auf der Auflagefläche 240 eine Tibiakomponente einer Knieendoprothese befestigt werden und die Sägelehre 100 kann durch Herausdrehen der Knochenschrauben 114, 115 entfernt werden.

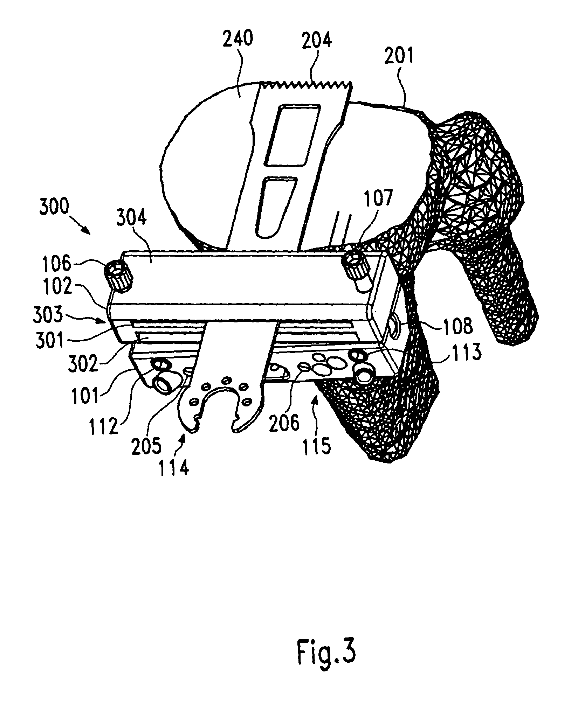

Fig. 3 zeigt eine schematische Perspektivansicht einer Sägelehre 300 gemäß einer weiteren Ausführungsform der vorliegenden Erfindung. Der Einfachheit halber wurden in Fig. 3 und in den Fig. 1, 2A, 2B und 2C gleiche Bezugszeichen verwendet, um gleiche Teile zu bezeichnen.

Die Sägelehre 300 umfasst einen Befestigungsabschnitt 101 mit mehreren ersten Bohrungen 114 und mehreren zweiten Bohrungen 115. Durch die mehreren ersten Bohrungen 114 und die mehreren zweiten Bohrungen 115 können Befestigungsmittel, z.B. Knochenschrauben 205, 206 geführt werden, um die Sägelehre 300 an einem Tibiaknochen 201 zu befestigen. Die Sägelehre 300 umfasst femer einen Sägeführungsabschnitt 102, der durch zwei Stellschrauben 106, 107 mit dem Befestigungsabschnitt 101 verbunden ist, wodurch eine Ausrichtung des Sägeführungsabschnitts 102 ermöglich wird. Die Stellschrauben 106, 107 können zueinander parallele Achsen aufweisen. Enden der Stellschrauben 106, 107 können durch Gelenke mit dem Befestigungsabschnitt 101 verbunden sein. Das Gelenk für die Schraube 107 ist in Fig. 3 durch das Bezugszeichen 108 bezeichnet. Der Sägeführungsabschnitt 102 kann zwei Führungsfüße (nicht gezeigt) aufweisen, die jeweils in Öffnungen im Befestigungsabschnitt 101 eingreifen. Die Führungsfüße des Befestigungsabschnitts 102 können durch Feststellschrauben 112, 113 im Befestigungsabschnitt 101 arretiert werden. Diese Merkmale können Merkmalen der oben mit Bezug auf die Fig. 1 bis 2c beschriebenen Ausführungsform entsprechen.

Der Sägeführungsabschnitt 102 umfasst Führungsmittel 303. Die Führungsmittel 303 umfassen einen ersten Schlitz 301 und einen zweiten Schlitz 302. Eine Breite der Schlitze 301, 302 ist derart ausgebildet, dass ein Sägeblatt 204 einer Knochensäge eines den Fachleuten bekannten Typs nach der Ausrichtung des Sägeführungsabschnitts 102 durch die Schlitze 301, 302 gesteckt werden kann, wobei sich das Sägeblatt 204 in einer Ebene, die durch die Orientierung des jeweiligen Schlitzes 301, 302 definiert ist, bewegen kann.

Durch Bewegen des Sägeblatts 204 innerhalb der durch einen der beiden Schlitze 301, 302 definierten Ebene kann ein Stück des Tibiaknochens 201 entfernt werden und so eine Auflagefläche 240 auf dem Tibiaknochen 201 erzeugt werden, auf der eine Tibiakomponente einer Knieendoprothese eines den Fachleuten bekannten Typs befestigt werden kann.

Die beiden Schlitze 301, 302 können unterschiedliche Abstände zum Befestigungsabschnitt 101 aufweisen. In manchen Ausführungsformen können die beiden Schlitze 301, 302 auch zueinander geneigt sein, beispielsweise in einer zur Längsrichtung der Schlitze 301, 302 senkrechten Tiefenrichtung der Schlitze 301, 302. Somit kann durch Auswahl eines der beiden Schlitze 301, 302 eine Position und/oder Neigung der Auflagefläche 240 eingestellt werden.

Die vorliegende Erfindung ist nicht auf Ausführungsformen beschränkt, in denen ein Führungsmittel, das zum Führen eines Sägeblatts in einer Ebene geeignet ist, mehrere Rundstäbe 104, 105, 116, 117 (Fig. 1 bis 2C) oder Schlitze 301, 302 (Fig. 3) umfasst. In anderen Ausführungsformen kann der Sägeführungsabschnitt 102 eine Auflagefläche aufweisen, auf der das Sägeblatt 204 aufgelegt werden kann. Die Auflagefläche kann eine ebene Fläche ähnlich der Oberfläche 304 der in Fig. 3 gezeigten Sägelehre 300 sein.

## Patentansprüche

1. Sägetehre (100) zum Durchführen einer Knochenresektion, umfassend:
einen Befestigungsabschnitt (101) mit mehreren Bohrungen (114, 115) zur Aufnahme von Befestigungsmitteln, die für eine Befestigung des Befestigungsabschnitts an einem Knochen ausgebildet sind;
einen Sägeführungsabschnitt (102), der ein Führungsmittel (103) umfasst, das zum Führen eines Sägeblatts (204) in einer Ebene geeignet ist,
**dadurch gekennzeichnet, dass**
der Sägeführungsabschnitt durch zwei Stellschrauben (106, 107) mit dem Befestigungsabschnitt verbunden ist, wobei das Führungsmittel zwischen den Stellschrauben angeordnet ist und wobei die Stellschrauben zueinander parallele Achsen aufweisen; und wobei ein Ende von jeder der Stellschrauben durch ein Gelenk (108) mit dem Befestigungsabschnitt verbunden ist.

2. Sägelehre nach Anspruch 1, in der jede der Bohrungen zur Aufnahme eines Knochennagels oder einer Knochenschraube ausgebildet ist.

3. Sägelehre nach Anspruch 1 oder 2, in der die Bohrungen zueinander im wesentlichen parallel sind.

4. Sägelehre nach einem der vorhergehenden Ansprüche, in der der Sägeführungsabschnitt zwei Führungsfüße (110, 111) aufweist, die jeweils in Öffnungen im Befestigungsabschnitt eingreifen, wobei die Sägelehre ferner zwei Feststellschrauben (112, 113) zum Arretieren der Führungsfüße im Befestigungsabschnitt umfasst.

5. Sägelehre nach einem der vorhergehenden Ansprüche, in der das Führungsmittel (103) mehrere zueinander parallele Rundstäbe (104, 105, 116, 117) umfasst.

6. Sägelehre nach einem der Ansprüche 1 bis 4, in der das Führungsmittel einen im Sägeführungsabschnitt (102) ausgebildeten Schlitz (301) umfasst.

7. Sägelehre nach einem der Ansprüche 1 bis 4, in der das Führungsmittel eine ebene Auflagefläche am Sägeführungsabschnitt umfasst.

## Claims

1. Saw jig (100) for carrying out a bone resection, comprising:
a fastening portion (101) having a plurality of holes (114, 115) for receiving fastening means which are adapted for fastening the fastening portion to a bone;
a saw guide portion (102) comprising a guide means (103) which is adapted for guiding a saw blade (204) in a plane,
**characterised in that**
the saw guide portion is connected to the fastening portion by two set screws (106, 107), the guide means being arranged between the set screws and the set screws having mutually parallel axes; and one end of each of the set screws being connected to the fastening portion by an articulation (108).

2. Saw jig according to claim 1, where in each of the holes is formed for receiving a bone nail or a bone screw.

3. Saw jig according to either claim 1 or claim 2, where in the holes are substantially mutually parallel.

4. Saw jig according to any one of the preceding claims, where in the saw guide portion comprises two guide feet (110, 111) which each engage in openings in the fastening portion, the saw jig further comprising two locking screws (112, 113) for locking the guide feet in the fastening portion.

5. Saw jig according to any one of the preceding claims, where in the guide means (103) comprises a plurality of mutually parallel round rods (104, 105, 116, 117).

6. Saw jig according to any one of claims 1 to 4, where in the guide means comprises a slot (301) formed in the saw guide portion (102).

7. Saw jig according to any one of claims 1 to 4, where in the guide means comprises a planar support surface on the saw guide portion.

## Revendications

1. Gabarit de sciage (100) pour effectuer une résection osseuse, comprenant :
une partie de fixation (101) avec plusieurs perçages (114, 115) destinés à recevoir des moyens de fixation qui sont conçus pour fixer ladite partie de fixation à un os ;
une partie de guidage de scie (102) qui comprend un moyen de guidage (103) apte à guider une lame de scie (204) dans un plan,
**caractérisé en ce que** la partie de guidage de scie est reliée par deux vis de réglage (106, 107) à la partie de fixation, le moyen de fixation étant disposé entre les vis de réglage, et les vis de réglage présentant des axes parallèles ; et une extrémité de chacune des vis de réglage étant reliée par une articulation (108) à la partie de fixation.

2. Gabarit selon la revendication 1, dans lequel chacun des perçages est conçu pour recevoir un clou à os ou une vis à os.

3. Gabarit selon la revendication 1 ou 2, dans lequel les perçages sont globalement parallèles.

4. Gabarit selon l'une des revendications précédentes, dans lequel la partie de guidage de scie présente deux pieds de guidage (110, 111) qui entrent dans des ouvertures de la partie de fixation, le gabarit de sciage comprenant par ailleurs deux vis de fixation (112, 113) pour arrêter les pieds de guidage dans la partie de fixation.

5. Gabarit selon l'une des revendications précédentes, dans lequel le moyen de guidage (103) comprend plusieurs tiges rondes (104, 105, 116, 117) parallèles.

6. Gabarit selon l'une des revendications 1 à 4, dans lequel le moyen de guidage comprend une fente (301) formée dans la partie de guidage de scie (102).

7. Gabarit selon l'une des revendications 1 à 4, dans lequel le moyen de guidage comprend une surface d'application plane sur la partie de guidage de scie.
